# EUROPEAN PATENT SPECIFICATION

(11) **EP 1 742 584 B1**
(45) Date of publication and mention of the grant of the patent: **25.08.2010**
(21) Application number: 05740393.3
(22) Date of filing: 06.04.2005
(51) Int. Cl.: A61B 17/17, A61B 19/00

(54) **AUTOMATIC POINTING DEVICE FOR CORRECT POSITIONING OF THE DISTAL LOCKING SCREWS OF AN INTRAMEDULLARY NAIL**
AUTOMATISCHE ZEIGERVORRICHTUNG FÜR DIE KORREKTE POSITIONIERUNG VON DISTALEN ARRETIERSCHRAUBEN EINES MARKNAGELS
DISPOSITIF DE POINTAGE AUTOMATIQUE PERMETTANT UN POSITIONNEMENT CORRECT DES VIS DE BLOCAGE DISTALES D'UN CLOU INTRAMEDULLAIRE

(30) Priority: 08.04.2004 IT MI20040695
(43) Date of publication of application: 17.01.2007
(73) Proprietor: Teleios S.R.L., 70122 Bari (IT)
(72) Inventor: ROSATI, Giorgio, 20178 ROMA (IT); SECCO, Lorenzo, 20178 ROMA (IT); RIZZO, Gaetano, I-70122 Bari (IT)
(74) Representative: Banfi, Paolo
(86) International application number: PCT/EP2005/003616
(87) International publication number: WO 2005/096961

(56) References cited:
- WO-A-03/043485
- US-A- 5 517 990
- US-A- 6 074 394
- US-A1- 2002 077 540
- US-B1- 6 285 902

## Description

This invention relates to an automatic pointing device for the correct positioning of the distal locking screws of an intramedullary nail, which exactly detects the position and inclination of the hole in the distal part of the nail so that a cutter guide element can be positioned accordingly.

In particular, the machine according to the invention includes means, mounted on the same structure, designed to position a reference in correspondence with the area in which the hole in the intramedullary nail is located; means designed to capture and process an X-ray image of the hole and the reference system; means designed to calculate the position of the hole and the angle of its axis in relation to said reference; means designed to position a cutter guide in axis with said hole, depending on the coordinates of the hole in relation to said reference, which are detected by said detection means; and means designed to calculate the length of the screw automatically.

With the apparatus according to the invention, the person performing the operation can precisely establish the position of the hole and position the cutter in axis with it, thus eliminating the currently rather frequent risk of imprecise positioning, with all the problems that entails.

The apparatus according to the invention may also measure the width of the bone in correspondence with the axis of the hole, thus enabling the surgeon to choose a screw of a length suitable for the type of operation to be performed.

The invention relates to the orthopaedic sector, and in particular to the stabilisation of fractures of the long bones.

According to the state of the art, three different systems are used to stabilise fractures of the long bones: plates and screws, external fastenings, and intramedullary nails.

Plates are constituted by metal elements which are attached to the bone through generally large surgical incisions, and secured with screws. They present the drawback that apart from rare exceptions, they involve highly invasive procedures, and weight cannot be placed on the bone at an early date.

External fastenings are devices that can be quickly implanted, only require small access holes for their fitting, and enable weight to be placed on the bone in a short time; however, they involve lengthy exposure of both doctor and patient to X-rays, and are hard to check to ensure perfect alignment of the fracture edges, especially in the case of a multifragmentary fracture.

Intramedullary nails are constituted by metal rods inserted into the bone, along its entire length, through small surgical incisions.

Although intramedullary nails are quick to implant, with good alignment of the bone edges even in the case of comminuted fractures, and allow early loading and recovery of the bone, they present the drawback of high exposure of the patient and operators to X-rays, mainly due to the considerable difficulty of precisely establishing the position of the holes into which the fixing screws are to be inserted.

The intramedullary nailing technique makes use of a characteristic common to all the long bones: the fact that they are hollow. This means that a metal rod of suitable length can be inserted into the bone, so that the parts take up a perfectly aligned position, thus allowing the fracture to heal correctly.

The first nails used in this technique were rigid, and had a cross-section which did not completely fill the medullary channel.

However, the bone edges were liable to twist or to slip along the nail, especially in multifragmentary fractures, because there was no load-bearing bone structure to withstand the axial loads. This led to shortening of the bone and rotation defects, with all the resulting consequences.

To eliminate this problem, intramedullary nails have been designed which are secured with screws that run through the bone and the nail from side to side, thus firmly anchoring the ends of the fractured bone and preventing them from sliding towards one another.

Subsequent developments in technology have led to the manufacture of elastic nails with a curved shape so that they adapt better to the anatomy of the bone; said nails are made of biocompatible metal, do not entirely fill the medullary channel, and are locked at the ends by screws that prevent the fractured bone from moving.

The surgical technique of intramedullary nailing currently involves aligning the fracture under the control of an X-ray apparatus and axially introducing the nail into the medullary channel through a small incision in the skin, again under the control of a fluoroscope or brightness amplifier, so that it runs through all fragments of the fracture.

When the nail has been positioned correctly, the system is rendered integral with the bone by means of locking screws inserted at the ends of the nail.

The screw closest to the point of insertion of the nail, namely the proximal extremity, is guided by a mechanical system of known type which enables the hole in the nail in the bone to be centred with precision.

However, the problem of correct positioning of the distal locking screw arises, because the point of insertion is difficult to determine due to the anatomical shape of the long bones, which are never straight, and the deformability of the metal used to make the nail.

External mechanical systems are consequently unable to reproduce the deformations undergone by the nail during positioning, and this creates great difficulties for the surgeon and causes a very high error rate.

To position the screws in the correct seating, the surgeon takes a number of images of the area with an X-ray apparatus and determines the point of entry of the screw on the basis of his experience.

However, this technique requires multiple pointing attempts which expose both the patient and the surgeon to a considerable quantity of radiation, and also has a very high error rate.

This happens because the image obtained with the X-ray apparatus is two-dimensional, and a three-dimensional image of the object can only be reconstructed, with experience, by combining a number of images taken from different angles in a process entirely based on the surgeon's calculation skills.

US patent no. 5,517,990 describes a guided-image surgical system; however, this system exposes both surgeon and patient to a high dose of radiation. Said system is also considered too difficult to use, and increases operating times excessively.

US patent 6,285,902 describes the use of pointing apparatus able to guide the operator's hand. Navigation is based on software that integrates X-ray images with anatomical findings identified by a stereoscopic system of video cameras. With the aid of these means, the operator can follow the movements of the surgical instruments on a screen.

This system uses the surgeon as an assistant to improve the result, instead of assisting the surgeon by supplying him with precise information to perform the procedure.

Document WO 03/043485 discloses a computer assisted surgery system for assisting a surgeon in aligning a drill with the interlocking holes of an implanted intramedullary rod used for fixation of long bone fractures. A localizing device measures the pose of a tracked adapter that is attached to the rod's exposed end and X-ray images are acquired of the end of the rod with the interlocking holes. Image processing algorithms determine the actual position of the rod and calculate an adjustment to the pose of the tracked adapter and rod. By using the adjusted pose information, the system is able to display a representation of the drill trajectory relative to the images of the rod and relative to a graphic representation of the rod.

Basically, no quick, reliable solution to the problem of correct positioning of the fixing screws of an intramedullary nail has yet been found, and the need is therefore felt in the industry for means which allow automatic operation, without forcing the surgeon to watch the monitor continually while he operates, thus providing a precise instrument which uses calculation processes independent of the quality of the X-ray images stored.

This problem is now solved by the present invention, which offers fully automatic pointing devices for the correct positioning, of the distal locking screws of an intramedullary nail.

The devices reconstruct the reciprocal coordinates and spatial orientation of the target and of the pointing device from two or more fluoroscopic, X-ray or other images, and on the basis of the data found automatically position a cutter guide, operated by the surgeon or activated in a servo-assisted manner, that serves to prepare the housing for the distal locking screws of the intramedullary nail, which said guide may be constituted, for example, by a cannula.

These devices consequently support the surgeon's action and experience without replacing it. It is therefore, unnecessay to change the operator's learning processes or operating procedures.

This and other purposes are achieved by devices in accordance with one or more of the annexed claims.

The invention is defined in the independent claim 1. Preferred embodiments are defined in the dependent claims.

This invention will now be described in detail, by way of example but not of limitation, by reference to the annexed examples and drawings wherein:
- figures 1 and 2 show an operating table of known type, equipped for normal orthopaedic operations, with a schematic representation of a first embodiment of an apparatus;
- figure 3 schematically illustrates the structure of the apparatus according to this first embodiment;
- figure 4 schematically illustrates the structure of the pointing devices in a apparatus according to this first embodiment;
- figure 5 is a flow chart which illustrates the operating procedure;
- figure 6 schematically illustrates the apparatus according to this first embodiment a whole, with a disposable protective hood;
- figures 7 and 8 show an operating table of known type, equipped for normal orthopaedic operations, with a schematic representation of a second embodiment;
- figure 9 schematically illustrates the structure of the apparatus according to said second embodiment;
- figure 10 schematically illustrates the structure of the pointing devices in said second embodiment;

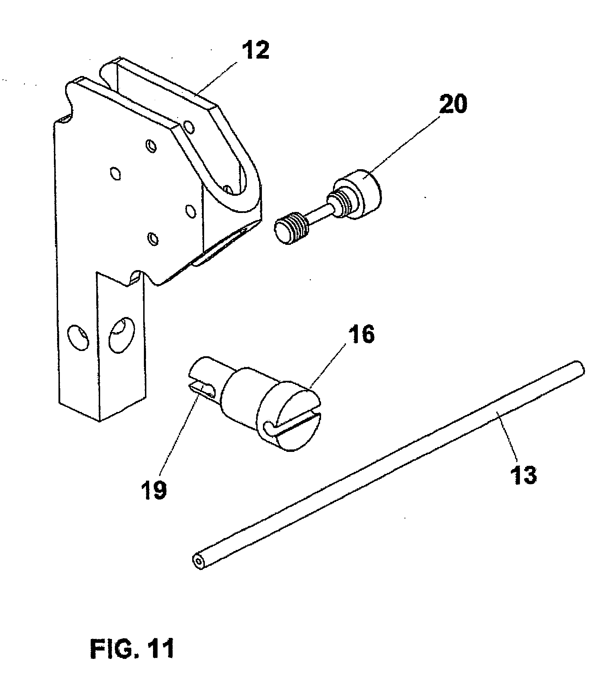

- figure 11 shows the details of the connection and locking system between the reference system and the cannula-holding terminal;
- figure 12 schematically illustrates the apparatus as a whole, with a disposable protective hood.

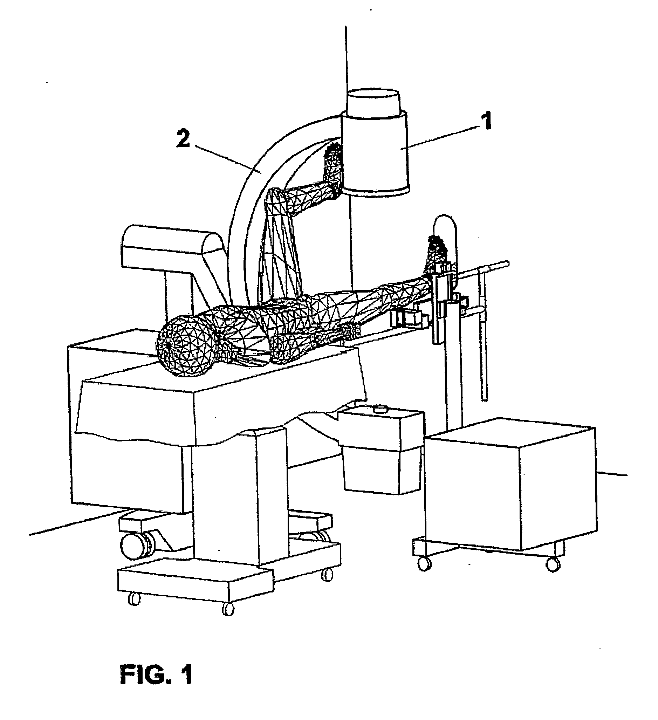

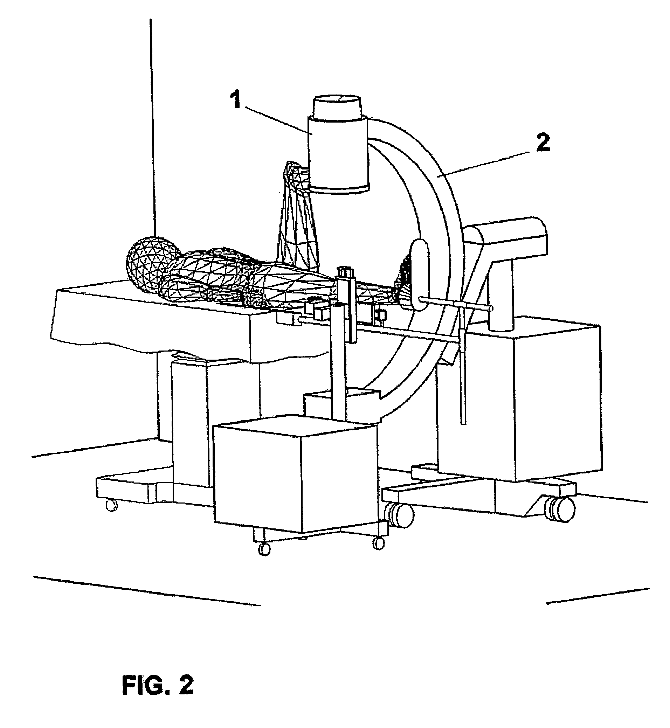

Figures 1 and 2 schematically illustrate an operating table equipped for orthopaedic operations, with which said apparatus according to the invention is associated.

The operating table is of known type, and there is no need to describe it. For the purpose of understanding this invention, it is sufficient to know that said table is equipped with X-ray, fluoroscopic or other apparatus 1, mounted on a C-shaped support 2, which allows said apparatus to be moved along an arc in order to perform X-rays according to planes inclined in relation to one another, such as orthogonal planes, said apparatus having an output, not illustrated in the figure, for an analogue or digital signal which allows the images taken by head 1 to be displayed on a computer or other similar apparatus.

Fluoroscopic, X-ray or other apparatus 1 is used to take two or more images of the distal extremity of the nail when it has been inserted in the bone, and to calculate the position and inclination of the hole in the nail for the purpose of subsequent insertion of the locking screws.

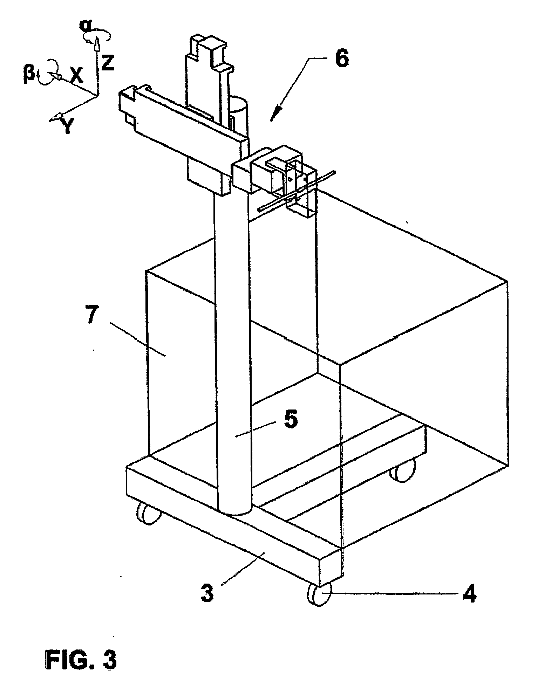

The apparatus according to this first aspect is schematically illustrated as a whole in figure 3, and comprises a base 3, mounted on wheels 4, which is integral with an upright 5 supporting a pointing system 6.

Electronic devices such as a computer or the like, schematically illustrated and indicated as 7, receive the images originating from fluoroscopic apparatus 1 as input, process them, and then control the positioning of pointing system 6.

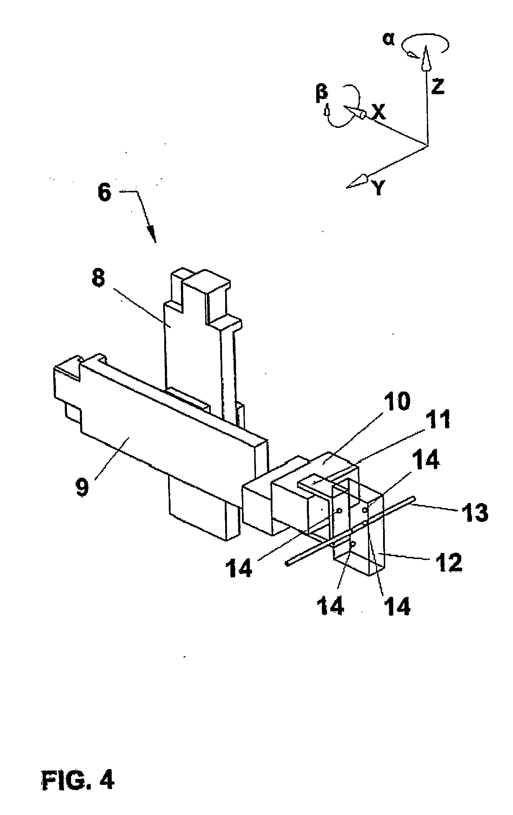

Said system, which is schematically illustrated and more clearly visible in figure 4, comprises a first slide 8, fitted to upright 5 in such a way that it can move along a vertical axis (axis Z) under numerical control, and a second slide 9, orthogonal to the preceding slide, which is mounted an the latter in such a way that it can move, again under numerical control, along a horizontal axis X which is orthogonal to the preceding axis.

The movements of the two slides are activated, for example, by means of stepping, motors which activate recirculating ball shafts or another known system.

A first support 10, which can rotate, again under numerical control, around an axis parallel to axis X, is fitted to slide 9.

A second support 11, which can rotate around a substantially vertical axis parallel to axis Z, is hinged to said first support 10.

The movement of support 11 is also activated by a stepping motor.

A possibly sterile head 12 is fitted to support 11 via a coupling of known type. A cannula 13, also possibly sterile, is inserted through head 12 and acts as a sight or pointing device to guide the cutting of the bone in correspondence with the holes in the nail when they have been located.

In accordance with one characteristic, the head also incorporates a reference system, constituted by three or more radio-opaque elements, and preferably by three or more spheres 14 made of metal or other suitable material, arranged in the shape of the vertices of a polygon, in the case illustrated in the figure in the shape of the vertices of two adjacent non-coplanar triangles.

This characteristic is of great interest, because it enables the apparatus to operate separately from the operating table and the X-ray apparatus, as a reference system can be connected with the pointing device, allowing precise calculation of the movements to be performed by the pointer so that it moves into axis with the hole in the nail.

Basically, this characteristic allows the machine to operate and correctly position the pointer with no need for other references, thus making it possible to use the apparatus in association with any other type of equipment and any existing type of operating table and X-ray apparatus, with no need for any modifications.

As the systems for the movement of slides 8 and 9 and supports 10 and 11, and the corresponding control software, are known, a detailed description thereof is not required.

Head 12 is made of suitably shaped, sterile radiolucent material, incorporates spheres 14, allows the fitting of cannula 13, and is equipped with a quick-release coupling system for connection to support 11. For example, said coupling could be constituted by a conical or prismatic shank which is snap-fitted into a corresponding seating in the support, to allow rapid fitting and/or replacement of the head.

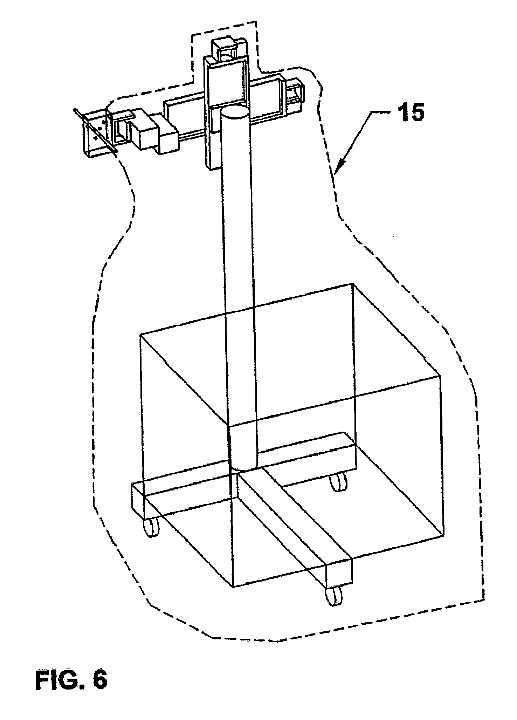

A pointing cannula is attached to the sterile head to guide the cutter or a wire over which a cannulated cutter of suitable gauge can be passed; a hood, also sterile, schematically illustrated in figure 6 as no. 15, is anchored to said head; said hood may be made of plastic, silicone, non-woven fabric or other disposable material, and is designed to be removed and eliminated at the end of the surgical operations.

Advantageously, head 12 is sterile and integral with the hood, which is rolled up, with only the shank projecting: when the shank has been inserted into the seating in support 11, the hood is opened and turned inside out so as to cover the entire apparatus, leaving exposed the head, onto which the guide cannula is fitted. Thus the head and the cannula are sterile, as is the other equipment in the proximity of the operating table.

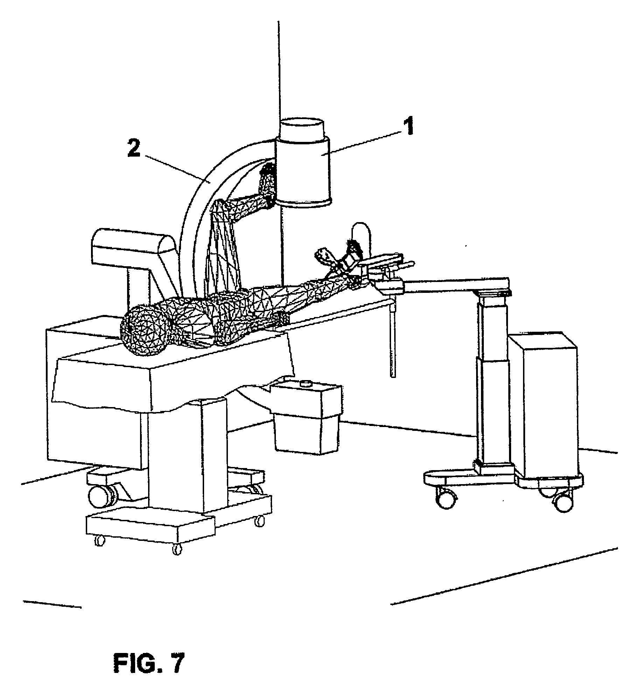

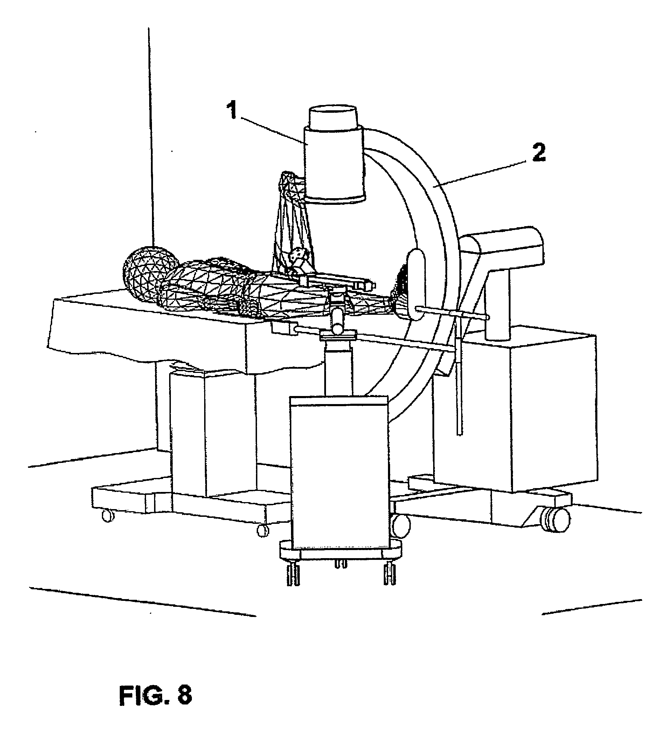

Figures 7 and 8 schematically illustrate an operating table equipped for orthopaedic operations, with which a second embodiment of the apparatus is associated.

As in the case of the preceding version, the operating table is of known type and there is no need to describe it. For the purpose of understanding this invention, it is sufficient to know that said table is equipped with X-ray, fluoroscopic or other apparatus 1, mounted on a C-shaped support 2, which allows said apparatus to be moved along an arc in order to perform X-rays according to planes inclined in relation to one another, such as orthogonal planes, said apparatus having an output, not illustrated in the figure, for an analogue or digital signal which allows the images taken by head 1 to be displayed on a computer or other similar apparatus 1.

The fluoroscopic, X-ray or other apparatus is used to take two or more images of the distal extremity of the nail when it has been inserted in the bone, and to calculate the position and inclination of the hole in the nail for the purpose of subsequent insertion of the locking screws.

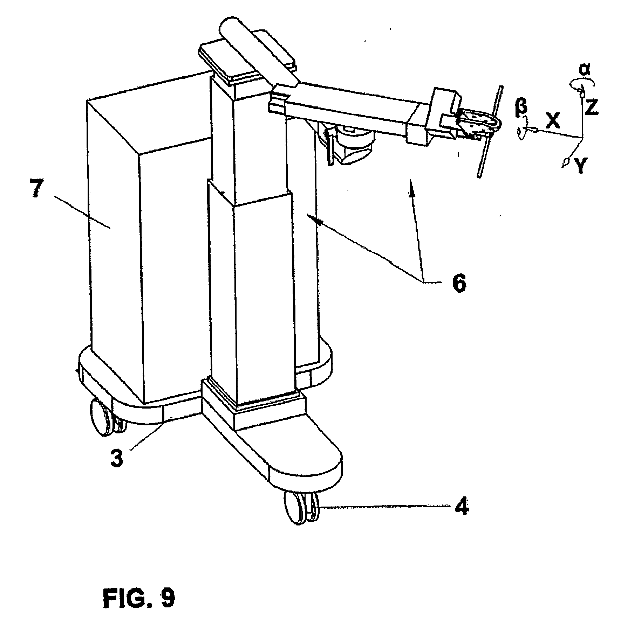

The apparatus is schematically illustrated as a whole in figure 9, and comprises a base 3, mounted on wheels 4, which is integral with a pointing system 6.

Electronic devices such as a computer or the like, schematically illustrated and indicated as 7, receive the images originating from X-ray, fluoroscopic or other apparatus 1 as input, process them, and then control the positioning of pointing system 6.

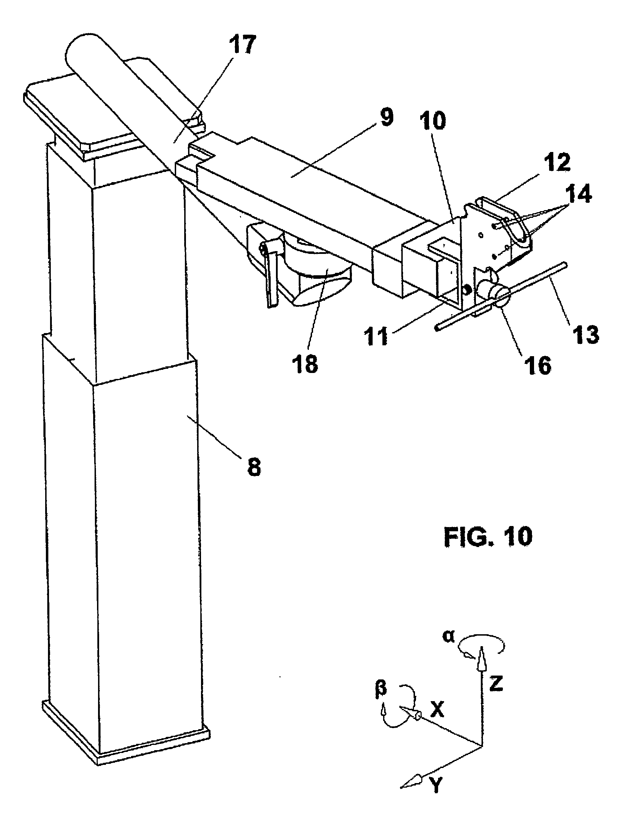

Said system, which is schematically illustrated and more clearly visible in figure 10, comprises an upright 8, which can move along a vertical axis (axis Z) under numerical control, and an arm 9, which is orthogonal to the preceding one and connected to it via a rigid cantilever 17 and a swivelling manual joint 18 which can move, again under numerical control, along a horizontal axis X which is orthogonal to the preceding axis Z.

Said upright and arm are activated, for example, by means of stepping motors which activate recirculating ball shafts or another known system.

A first support 10, which can rotate, again under numerical control, around an axis parallel to axis X, is fitted to arm 9.

A second support 11, which can rotate around a substantially vertical axis parallel to axis Z, is hinged to said first support 10.

The movement of support 11 can also be activated by a stepping motor. In accordance with one characteristic, a head 12, which incorporates a reference system constituted by three or more radiopaque elements, and preferably by three or more spheres 14 made of metal or other suitable material, arranged in the shape of the vertices of a polygon, and in the case illustrated in the figure in the shape of the vertices of two adjacent non-coplanar triangles, is fitted to support 11 via a fastening af known type (such as screws).

Said variation on the reference system is characterised in that the radiopaque spheres are staggered in relation to the rotation of support 10 on which the head is fitted, in such a way that when support 10 is rotated, the possibility that the reference system will be superimposed with the intramedullary nail in the X-ray, fluoroscopic or other images is minimised, thus facilitating correct imaging of the spheres and the target holes in the intramedullary nail.

The coupling system of sterile terminal 16 is fitted to head 12, which contains the radiopaque spheres. A guide such as cannula 13, also sterile, is inserted through terminal 16 and acts as a sight or pointing device to guide the cutting of the bone in correspondence with the holes in the nail when they have been located.

The reference system with radiopaque elements arranged in the shape of the vertices of a polygon, as described above, is of great interest, because it enables the apparatus to operate separately from the operating table and the X-ray, fluoroscopic or other apparatus, because a reference system can be connected with the pointing device, allowing precise calculation of the movements to be performed by the pointer so that it moves into axis with the hole in the nail.

Basically, this reference system enables the machine to operate and correctly position the pointer with no need for other references, thus making it possible to use the apparatus in association with any other type of equipment and any type of existing operating table and X-ray apparatus, without any need for modifications.

As the systems for the movement of slides 8 and 9 and supports 10 and 11, and the corresponding control software, are known, a detailed description thereof is not required.

Terminal 16 has a suitable shape to allow the fitting of cannula 13, and has a shank 19 designed to be inserted into a hole in a wall of head 12. Shank 19 has a groove into which the shank of a double-coned screw 20 is inserted to lock terminal 16 into position on head 12.

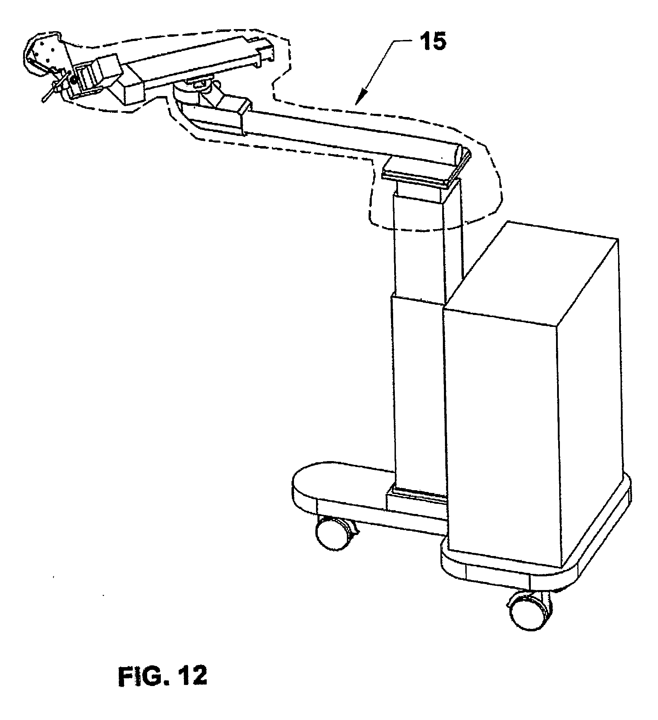

Terminal 16 allows the insertion of a pointing cannula which acts as guide for the cutter or a metal wire (such as a Kirshner wire) onto which a cannulated cutter of suitable gauge is passed. A hood 15, also sterile, which is schematically illustrated in figure 12, may be anchored to the same head; said hood can be made of plastic, silicone, non-woven fabric or another disposable material, and is destined to be removed and eliminated when the surgical operations are finished.

Advantageously, terminal 16 can be sterile and integral with the hood, which is rolled up, with only the shank projecting: when the shank has been inserted into the seating in support 10 in the first embodiment or the seating in system 12 in the second embodiment, the hood is opened and turned inside out so that it covers the entire apparatus, leaving exposed terminal 16, on which the cannula guide is fitted. Thus the terminal and the cannula are sterile, as is the other equipment in the proximity of the operating table.

The apparatus described operates as follows.

When the nail has been inserted into the bone and its proximal end secured (so far according to the prior art), the surgeon prepares the equipment to secure the distal extremity with screws.

For this purpose the surgeon brings the apparatus close to the operating table, in proximity with the area in which the holes are to be made for the insertion of the screws, by manoeuvring carriage 3 and upright 5 in embodiment 1 or system 6 in embodiment 2, both of which swivel on wheels 4.

At this point, two images of the end of the nail are taken with X-ray, fluoroscopic or other apparatus 1, which can slide on C-shaped arm 2; said images are taken according to different planes, preferably two orthogonal planes, taking care also to show head 12 which incorporates the reference constituted by spheres 14.

Said X-rays can be taken without the medical personnel having to remain close to the radiography apparatus.

The X-ray system supplies two images which show the end of the nail, with the holes and the reference constituted by spheres 14.

Said images are sent to computer 7 for processing.

Depending on the filming angle, the holes in the nail may not appear (when the images are taken according to an axis perpendicular to the axis of the hole), appear as a circle (when the images are taken exactly in axis with the hole), or appear as the intersection of two elliptical arches (for all the intermediate positions).

The geometrical characteristics of the X-rayed nail are known and stored in a database available to the software system. Consequently, the system knows the exact shaps, dimensions and position of the holes for distal fixing of each type of nail which can be used, and the characteristics of reference 14 incorporated in the head (mutual positions and diameter of the spheres). On the basis of the information obtained from software processing of the images, the system calculates the mutual positions and orientation of the reference and the axis of the target hole.

Comparative analysis of the perspective deformation of a hole and of a reference with known dimensions and geometries between at least two X-ray images taken simultaneously allows the angles at which the images were taken to be determined, so that a three-dimensional reconstruction of their relative positions and orientation can be performed, as stated above.

As the position of the cannula in relation to the reference is known, the movement of the cannula required to align it with the axis of the hole can be determined; the movement is then broken down according to the four degrees of freedom allowed by the linear slides and the head so that it can reach the new position. Finally, the computer sends the movement data to the controlled responsible for moving the slides and the terminal, thus positioning the cannula in axis with the target hole.

By controlling upright 8 and arm 9 and the rotation of supports 10 and 11, as identified in both examples, the machine brings the hole in head 12 in embodiment 1 or in terminal 16 in embodiment 2 into axis with the hole in the nail. It also automatically calculates the optimum length of the distal screws, as will be explained below.

The surgeon then attaches sterile cannula 13 to the terminal and positions a metal wire (e.g. a Kirshner wire) which acts as a guide for the cannulated cutter, enabling the surgeon to cut the hole in the bone with the maximum precision. Alternatively, the surgeon can cut the hole directly with the cutter.

Basically, therefore, the machine according to both illustrated embodiments takes images of the hole and spheres 14 and processes the dimensions and deformations of their geometries to calculate the mutual positions and inclination between the axis of the hole in the nail and the axis of the hole through which the cannula is inserted in head 12 in embodiment 1 or in terminal 16 in embodiment 2.

When the coordinates of the hole and the terminal are known, embodiments 1 and 2 of the machine calculate the movements of the various actuators in order to move the pointer into axis with the hole in the nail.

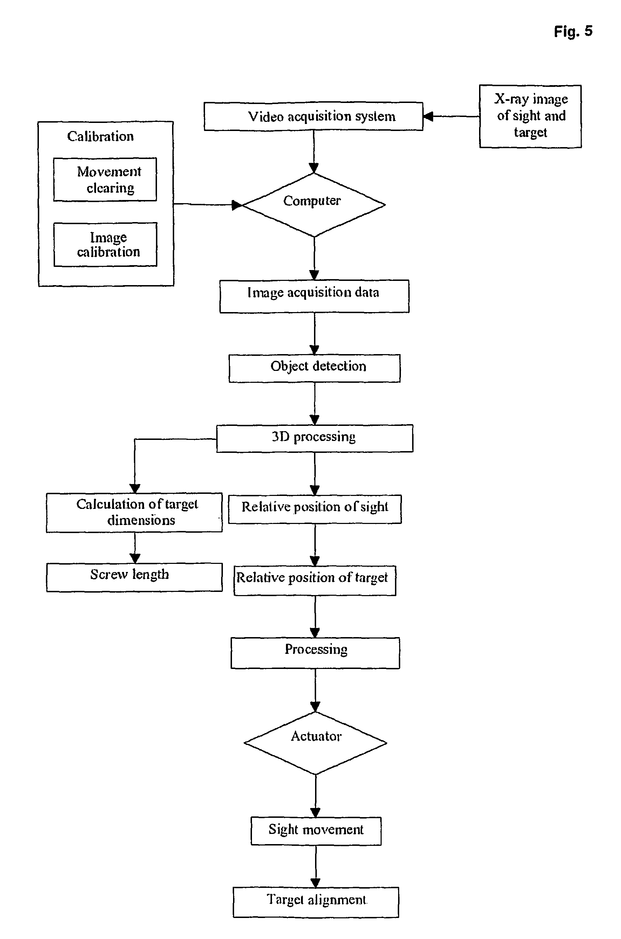

The flow chart in figure 5 illustrates the various stages of the operation.

The purpose is to determine the centre of gravity P (X, Y, Z) of the hole and the corresponding inclination vector.

A first latero-lateral image (plane X Z) and a second antero-posterior image (plane X Y) are taken.

The machine performs a first detection of the reference, identifying a Cartesian reference system of the reference (O' X' Y').

This is followed by detection of the hole. From the first image, the machine determines:
the orientation of the nail on a plane X Z (angle on Y), the definition of the pixel/mm ratio on the outer diameter of the nail or on another known, recognisable geometrical characteristic of the nail or the reference;
detection of the hole on the basis of the measurements of the two axes of the ellipse and consequent detection of the inclination of the axis of the hole on the plane X Z;
detection of the coordinates XP ZP on plane XZ.

From the second image, the machine determines:
the orientation of the nail on the plane XY (angle Z)
the definition of the pixel/mm ratio on the outer diameter of the nail;
and the detection of XP YP on plane XY.

On the basis of these values, by applying suitable algorithms of known type the machine determines the position of the axis of the hole in relation to that of reference 14, and can thus control the movements of the terminal to move it into axis.

The apparatus also automatically calculates the optimum length of the distal screws, by detecting the intersection of the line passing through the centre of the hole in the intramedullary nail and the bone cortex.

As well as knowing the precise point at which the hole is to be made, the surgeon therefore also receives the information required to choose the dimensions of the screw, according to the procedure he uses for the operation.

When the operation is finished, hood 15, head 12 or terminal 16, and guide 13 (such as a cannula) can be sent for sterilisation prior to subsequent reuse, or thrown away in the case of disposable equipment.

The apparatus according to the invention can be adapted for use with any intramedullary nail whose physical characteristics are included in its database.

## Claims

1. An automatic pointing apparatus for the correct positioning of the distal locking screws in respective holes of an intramedullary nail, said automatic pointing apparatus comprising:
• an automatic pointing system (6) comprising:
- a head (12) which includes:
- a reference system (14) designed to be viewed by an X-ray, fluoroscopic or similar apparatus (1), said reference system (14) being integral with said head (12) and being constituted by one or more radiopaque bodies (14) of known shape, dimensions and position, incorporated in said head (12), and
- means (13) of guiding a surgical instrument;
- means (8, 9, 10, 11; 17, 18) designed to automatically move said head (12) close to an end of the intramedullary nail containing said holes, so as to allow said X-ray, fluoroscopic or similar apparatus (1) to take simultaneous images of the end of the intramedullary nail with the holes and the reference system (14); and
• means (7) designed to:
- read said images and calculate the position and inclination of the axis of said holes on the basis of the shape and dimensions of the holes shown in the images;
- read the images of said reference system and calculate the relative position and inclination of said reference system, and consequently of said head (12), on the basis of the shape and dimensions of said reference system, and
- calculate the position and relative inclination of the axis of the holes in relation to said reference system and automatically move said head (12) through the means (8, 9, 10, 11; 17, 18) designed to move said head (12) so as to bring said means (13) of guiding the surgical instrument into axis with said holes, with the same inclination as the axis of the holes.

2. The automatic pointing apparatus as claimed in claim 1, **characterised in that** the head (12) includes a terminal (1b) designed to support the means (13) of guiding the surgical instrument.

3. The automatic pointing apparatus as claimed in claim 1 or 2, wherein said means (7) designed to read the images taken by said X-ray, fluoroscopic or similar apparatus (1) are constituted by a sensor connected via an interface to the output of said external X-ray, fluoroscopic or similar apparatus (1).

4. The automatic pointing apparatus as claimed in claim 2, wherein the reference system is separate from the terminal (16) and said terminal (16) is designed to be connected to the reference system via a quick-release coupling.

5. The automatic pointing apparatus as claimed in one or more of the preceding claims, **characterised in that** said one or more radiopaque bodies (14) are spheres.

6. The automatic pointing apparatus as claimed in one or more of the preceding claims, **characterised in that** said one or more radiopaque bodies (14) are located at the vertices of polygons of known dimensions.

7. The automatic pointing apparatus as claimed in any of the preceding claims, **characterised in that** the head (12) and the reference system are mounted on supports (10, 11) comprising a plurality of numerically controlled actuators designed to control the movements of the head (12) and the reference system according to at least two linear directions (X, Y, Z) orthogonal to one another, and their rotation (α, β) around at least two non-parallel axes.

8. The automatic pointing apparatus as claimed in claim 7, **characterised in that** it includes a sterile hood (15), fitted to said head (12), which is designed to cover the supports (10, 11) and any other parts which come into contact with the operating field.

## Patentansprüche

1. Automatische Zeigevorrichtung für die korrekte Positionierung von distalen Halteschrauben in entsprechenden Löchern eines intramedullaren Nagels, wobei die automatische Zeigevorrichtung umfasst:
ein automatisches Zeigesystem (6), umfassend:
einen Kopf (12), der enthält:
ein Bezugssystem (14), das ausgelegt ist, um durch eine Röntgenvorrichtung, Fluoreszenzvorrichtung oder eine ähnliche Vorrichtung (1) betrachtet zu werden, wobei das Bezugssystem (14) mit dem Kopf (12) eine Einheit bildet und durch einen oder mehrere strahlungsundurchlässige Körper (14) von bekannter Form, bekannten Abmessungen und bekannter Position gebildet wird, die in diesem Kopf (12) integriert sind, und
Mittel (13) zum Führen eines chirurgischen Instruments;
Mittel (8, 9, 10, 11; 17, 18), die ausgelegt sind, um den Kopf (12) automatisch dicht an ein Ende des intramedullaren Nagels zu bewegen, der die Löcher enthält, um es somit der Röntgenvorrichtung, Fluoreszenzvorrichtung oder ähnlichen Vorrichtung (1) zu ermöglichen, simultan Bilder des Endes des intramedullaren Nagels mit den Löchern und des Bezugssystems (14) aufzunehmen; und
Mittel (7), ausgelegt um:
die Bilder zu lesen und die Position und die Neigung der Achse der Löcher auf der Basis der Form und der Abmessungen der Löcher zu berechnen, die in den Bildern gezeigt sind;
die Bilder des Bezugssystems zu lesen und die relative Position und Neigung des Bezugssystems und folglich des Kopfes (12) auf der Basis der Form und der Abmessungen des Bezugssystems zu berechnen, und
die Position und die relative Neigung der Achse der Löcher bezüglich des Bezugssystems zu berechnen und den Kopf (12) durch die Mittel (8, 9, 10, 11, 17, 18), die ausgelegt sind um den Kopf (12) zu bewegen, automatisch zu bewegen, um somit die Mittel (13) der Führung des chirurgischen Instruments in Achse mit den Löchern, mit der gleichen Neigung wie die Achse der Löcher zu bringen.

2. Automatische Zeigevorrichtung gemäß Anspruch 1, **dadurch gekennzeichnet, dass** der Kopf (12) eine Endeinrichtung (16) enthält, die ausgelegt ist, um die Mittel 13) zum Führen des chirurgischen Instruments zu halten.

3. Automatische Zeigevorrichtung gemäß Anspruch 1 oder 2, wobei die Mittel (7), die ausgelegt sind, um die Bilder zu lesen, die durch die Röntgenvorrichtung, Fluoreszenzvorrichtung oder ähnlichen Vorrichtung (1) aufgenommen wurden, durch einen Sensor gebildet werden, der über eine Schnittstelle mit dem Ausgang der externen Röntgenvorrichtung, Fluoreszenzvorrichtung oder ähnlichen Vorrichtung (1) verbunden ist.

4. Automatische Zeigevorrichtung gemäß Anspruch 2, wobei das Bezugssystem von der Endeinrichtung (16) getrennt ist, und die Endeinrichtung (16) ausgelegt ist, um mit dem Bezugssystem über eine Schnellkupplung verbunden zu werden.

5. Automatische Zeigevorrichtung gemäß einem oder mehreren der vorhergehenden Ansprüche, **dadurch gekennzeichnet, dass** der eine oder die mehreren strahlungsundurchlässigen Körper 14) Kugeln sind.

6. Automatische Zeigevorrichtung gemäß einem oder mehreren der vorhergehenden Ansprüche, **dadurch gekennzeichnet, dass** der eine oder die mehreren strahlungsundurchlässigen Körper (14) an den Ecken von Polygonen bekannter Abmessungen platziert sind.

7. Automatische Zeigevorrichtung gemäß einem der vorhergehenden Ansprüche, **dadurch gekennzeichnet, dass** der Kopf (12) und das Bezugssystem an Haltern (10, 11) montiert sind, die mehrere numerisch-gesteuerte Aktuatoren umfassen, welche ausgelegt sind, um die Bewegungen des Kopfes (12) und des Bezugssystems gemäß mindestens zwei linearer Richtungen (X, Y, Z) zu steuern, die zueinander orthogonal sind, und ihre Rotation (α, β) um mindestens zwei nicht-parallele Achsen zu steuern.

8. Automatische Zeigevorrichtung gemäß Anspruch 7, **dadurch gekennzeichnet, dass** sie eine sterile Abdeckhaube (15) enthält, die an dem Kopf (12) angebracht und ausgelegt ist, um die Halter (10, 11) und alle andere Teile zu bedecken, die in Kontakt mit dem Arbeitsbereich kommen.

## Revendications

1. Dispositif de pointage automatique permettant un positionnement correct des vis de blocage distales dans les trous respectifs d'un clou intramédullaire, ledit dispositif de pointage automatique comprenant:
• un système de pointage automatique (6) comprenant :
- une tête (12) qui comprend:
- un système de référence (14) conçu pour être visualisé par un dispositif à rayons X, fluoroscopique ou similaire (1), ledit système de référence (14) faisant partie intégrante de ladite tête (12) et étant constitué d'un ou plusieurs corps radio-opaque(s) (14) ayant une forme, des dimensions et une position connues, intégré(s) à ladite tête (12), et
- un moyen (13) de guidage d'un instrument chirurgical ;
- des moyens (8, 9, 10, 11 ; 17, 18) conçus pour déplacer automatiquement ladite tête (12) près d'une extrémité du clou intramédullaire contenant lesdits trous, de façon à permettre audit dispositif à rayons X, fluoroscopique ou similaire (1) de prendre des images simultanées de l'extrémité du clou intramédullaire avec les trous et le système de référence (14) ; et
• un moyen (17) conçu pour :
- lire lesdites images et calculer la position et l'inclinaison de l'axe desdits trous sur la base de la forme et des dimensions des trous affichés sur les images ;
- lire les images dudit système de référence et calculer la position relative et l'inclinaison dudit système de référence, et, par conséquent, de ladite tête (12), sur la base de la forme et des dimensions dudit système de référence, et
- calculer la position et l'inclinaison relative de l'axe des trous par rapport audit système de référence et déplacer automatiquement ladite tête (12) à l'aide des moyens (8, 9, 10, 11 ; 17, 18) conçus pour déplacer ladite tête (12) de façon à amener ledit moyen (13) de guidage de l'instrument chirurgical dans l'axe desdits trous, avec la même inclinaison que l'axe des trous.

2. Dispositif de pointage automatique selon la revendication 1, **caractérisé en ce que** la tête (12) comprend un plot (16) destiné à supporter le moyen (13) de guidage de l'instrument chirurgical.

3. Dispositif de pointage automatique selon la revendication 1 ou 2, dans lequel ledit moyen (7) conçu pour lire les images prises par ledit dispositif à rayons X, fluoroscopique ou similaire (1) est constitué d'un capteur relié via une interface à la sortie dudit dispositif externe à rayons X, fluoroscopique ou similaire (1).

4. Dispositif de pointage automatique selon la revendication 2, dans lequel le système de référence est séparé du plot (16) et ledit plot (16) est conçu pour être relié au système de référence via un raccord à dégagement rapide.

5. Dispositif de pointage automatique selon une ou plusieurs des revendications précédentes, **caractérisé en ce que** ledit ou lesdits corps radio-opaque(s) (14) est/sont des sphères.

6. Dispositif de pointage automatique selon une ou plusieurs des revendications précédentes, **caractérisé en ce que** ledit ou lesdits corps radio-opaque(s) (14) est/sont situé(s) au niveau des sommets de polygones de dimensions connues.

7. Dispositif de pointage automatique selon l'une quelconque des revendications précédentes, **caractérisé en ce que** la tête (12) et le système de référence sont montés sur des supports (10, 11) comprenant une pluralité d'actionneurs à commande numérique conçus pour contrôler les mouvements de la tête (12) et du système de référence selon au moins deux directions linéaires (X, Y, Z) orthogonales l'une par rapport à l'autre, et leur rotation (α, β) autour d'au moins deux axes non parallèles.

8. Dispositif de pointage automatique selon la revendication 7, **caractérisé en ce qu'**il comprend un capot stérile (15), placé sur ladite tête (12), qui est conçu pour couvrir les supports (10, 11) et toute autre partie qui vient en contact avec le champ opératoire.
